(19) Europäisches Patentamt

European Patent Office

Office européen des brevets

(11) **EP 4 670 611 A1**

(12) **EUROPEAN PATENT APPLICATION**

(43) Date of publication:
**31.12.2025   Bulletin 2026/01**

(21) Application number: **24208032.3**

(22) Date of filing: **22.10.2024**

(51) International Patent Classification (IPC):
**A61B 1/00** *(2006.01)*        **G06T 5/20** *(2006.01)*
**G06T 5/73** *(2024.01)*

(52) Cooperative Patent Classification (CPC):
**G06T 5/73; A61B 1/000094; A61B 1/000095;
A61B 1/00082; G06T 5/20;** A61B 1/00103;
A61B 1/00105; G06T 2207/10068;
G06T 2207/20192; G06T 2207/30101

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **27.06.2024   CN 202410874256**

(71) Applicant: **Shenzhen Earchon Bio-Technique Co.
Ltd.
Shenzhen (CN)**

(72) Inventors:
• **Huang, Rongfeng
  Zhangshu City, Jiangxi Province (CN)**

• **Chen, Wei
  Wuhan (CN)**
• **Liu, Dongmei
  Zhangshu City, Jiangxi Province (CN)**
• **Zhang, Xianglei
  Shenzhen, Guangdong (CN)**
• **Xie, Hongji
  Lianping County, Guangdong Province (CN)**
• **Chen, Shumei
  Shenzhen, Guangdong (CN)**

(74) Representative: **Cleanthous, Marinos
  IOANNIDES CLEANTHOUS AND CO LLC
  4 Prometheus Street, 3 Floor
  1065 Nicosia (CY)**

(54) **ELECTRONIC FLEXIBLE AND RIGID ENDOSCOPE SYSTEM AND METHOD APPLICABLE TO GENERAL PRACTICE**

(57)    The present invention relates to an electronic flexible and rigid endoscope system and method applicable to general practice. The system comprises: a data acquisition system, configured to acquire image data of a lesion site, and a data processing system, configured to process the image data from the data acquisition system. The data acquisition system comprises a handle end and an endoscope end, the endoscope end comprises an imaging insertion end and an operating end, the imaging insertion end enters the lesion site to acquire images of the lesion site, the operating end controls the action of the imaging insertion end, the handle end sends the image data acquired by the endoscope end to the data processing system, the data processing system comprises a data receiving module and an image processing module, and the data receiving module receives the image data acquired by the data acquisition system.

```
                                                    ┌─ S10
     ┌─────────────────────────────────────────┐
     │      Acquiring image data of a lesion site │
     └─────────────────────────────────────────┘
                         │
                         │                          ┌─ S20
                         ▼
     ┌─────────────────────────────────────────┐
     │   Processing image data through an image dehazing │
     │   technology and a vascular enhancement technology, │
     │   wherein real-time dehazing is performed through a dark │
     │   channel dehazing algorithm, and blood vessels are │
     │   enhanced through a multi-scale vascular enhancement │
     │   algorithm, so that image processing is achieved │
     └─────────────────────────────────────────┘
```

FIG. 1

EP 4 670 611 A1

**Description**

**TECHNICAL FIELD**

The present invention relates to the field of electronic endoscopes, and in particular, to an electronic flexible and rigid endoscope system and method applicable to general practice.

**BACKGROUND**

A medical endoscope is a medical instrument that is inserted into a hollow organ or cavity of a human body and can examine the hollow organ and cavity of the human body without performing direct surgery. The medical endoscope can penetrate deep into the body cavity of a patient for diagnosis. This medical instrument is intuitive, reliable, and minimally invasive, and has become an irreplaceable diagnostic and treatment technology in clinical practice at present. The market for traditional optical endoscopes is largely occupied abroad. In recent years, the electronic endoscope examination and surgery have developed rapidly, which has surpassed the traditional optical endoscopes more and more rapidly in terms of cost, materials, and imaging quality.

Existing traditional endoscopes and some electronic endoscopes are mostly reusable, which need to withstand disinfection procedures (pressure resistance, high temperature resistance, etc.) and require a large amount of logistical support such as cleaning, disinfection, and drying. The cost is high, and cross infection may also occur due to incomplete disinfection. Meanwhile, existing endoscopes usually adopt a one-machine-one-scope mode, with a variety of equipment, resulting in a certain waste of resources. In addition, compared with traditional endoscopes, existing electronic endoscopes are inferior to traditional optical endoscopes in high-quality imaging. Traditional optical surgical endoscopes can achieve 4K resolution, but electronic medical endoscopes have failed to achieve ultra-high-definition applications.

**SUMMARY**

Aiming at the above defect in the prior art, an electronic flexible and rigid endoscope system and method applicable to general practice are provided.

To solve the above technical problem, the present invention adopts the following technical solutions.

An electronic flexible and rigid endoscope system applicable to general practice is constructed, which comprises: a data acquisition system, configured to acquire image data of a lesion site, and a data processing system, configured to process the image data from the data acquisition system, wherein the data acquisition system comprises a handle end and an endoscope end, the endoscope end comprises an imaging insertion end and an operating end, the imaging insertion end enters the lesion site to acquire images of the lesion site, the operating end controls the action of the imaging insertion end, the handle end sends the image data acquired by the endoscope end to the data processing system, the data processing system comprises a data receiving module and an image processing module, the data receiving module receives the image data acquired by the data acquisition system, and the image processing module performs dehazing through a dark channel prior algorithm and performs vascular enhancement through a multi-scale vascular enhancement algorithm.

The present invention has the beneficial effects that: the image processing system ensures that the acquired image is clear and real by adopting various image processing algorithms, which avoids misdiagnosis. Through the design of disposable endoscope head end, the disposable head end integrates a flushing channel, an instrument channel, a balloon and other designs, which solves the problems of small observation area, low observation limitation and complex operation, avoids multiple examinations and the waste of time and manpower and secondary injuries, making the examination more comprehensive and the operation easier. Meanwhile, a disposable design is applied, so that cross infection is avoided. The disposable head end design of multiple specifications can meet the examination and surgical scenarios of different departments, thus greatly reducing the cost of use. The reusable handle end uses a wireless design, and wireless data transmission makes the device lighter and frees the device from the constraints of wires. Meanwhile, the multifunctional camera terminal is separated into a reusable handle end and a disposable endoscope head end. The disposable head end can be used and thrown immediately, different instruments and flushing channels are designed based on the use scenarios, and an innovative balloon design is added to expand the observation area for better observation of lesions.

**BRIEF DESCRIPTION OF DRAWINGS**

FIG. 1 is a schematic structural diagram of a flexible and rigid endoscope system according to a preferred embodiment of the present invention;

FIG. 2 is a flow chart of a flexible and rigid endoscope method according to a preferred embodiment of the present invention;

FIG. 3 is a schematic diagram of a front structure of an endoscope end according to a preferred embodiment of the present invention; and

FIG. 4 is a schematic diagram of a back structure of an endoscope end according to a preferred embodiment of the present invention.

## DETAILED DESCRIPTION OF EMBODIMENTS

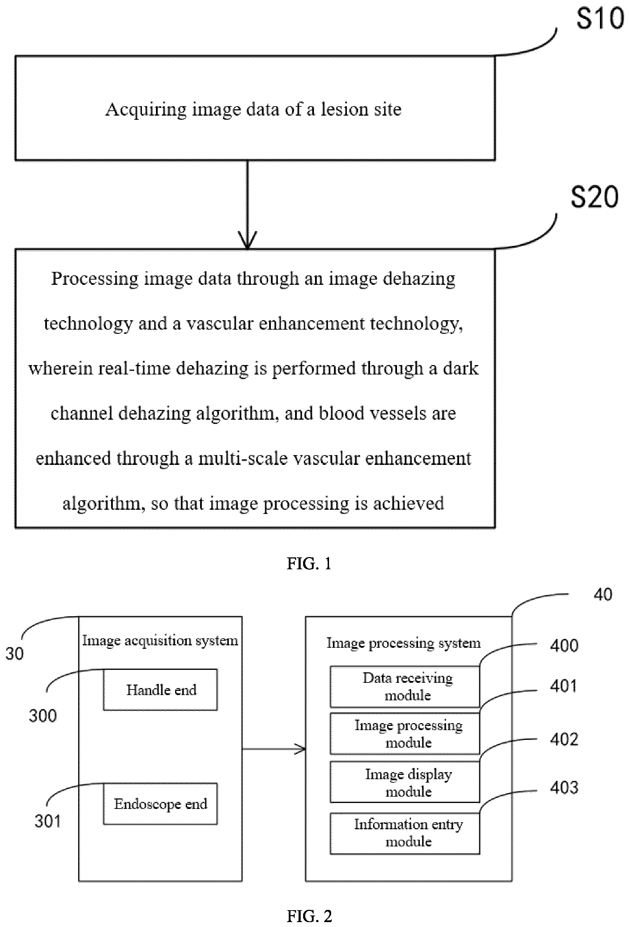

An electronic flexible and rigid endoscope system applicable to general practice according to a preferred embodiment of the present invention, as shown in FIG. 1, comprises an image acquisition system 30, and an image processing system 40 connected to the image acquisition system. A lesion site of a patient is imaged by the image acquisition system to generate image data, and the image data is sent to the data processing system, the data processing system processes and analyzes the acquired image data and then displays and stores the analyzed image data.

Specifically, as shown in FIG. 1, the image acquisition system 30 may adopt a multifunctional camera terminal, which comprises a handle end 300 and an endoscope end 301, and the reusable handle end is equipped with digital decoders for multiple lens models, an electronic light source control system, a battery management system and a wireless data transmission system, and the reusable handle end is controlled by embedded hardware and software. The digital decoder can quickly decode and encode data of various camera chips and convert the data into a universal digital signal so as to obtain a real and real-time endoscopic image. The electronic light source control system can adjust the brightness based on a requirement, and the brightness is divided into 4 levels, namely 0%, 33%, 66% and 100%. The wireless data transmission system uses 5G wireless transmission, which comprises H.265/HEVC video coding technology, which provides higher compression rate and maintains image quality. Meanwhile, the 5G band uses a high-frequency band, namely millimeter wave, with a frequency above 24 GHz, and the data transmission efficiency is extremely high.

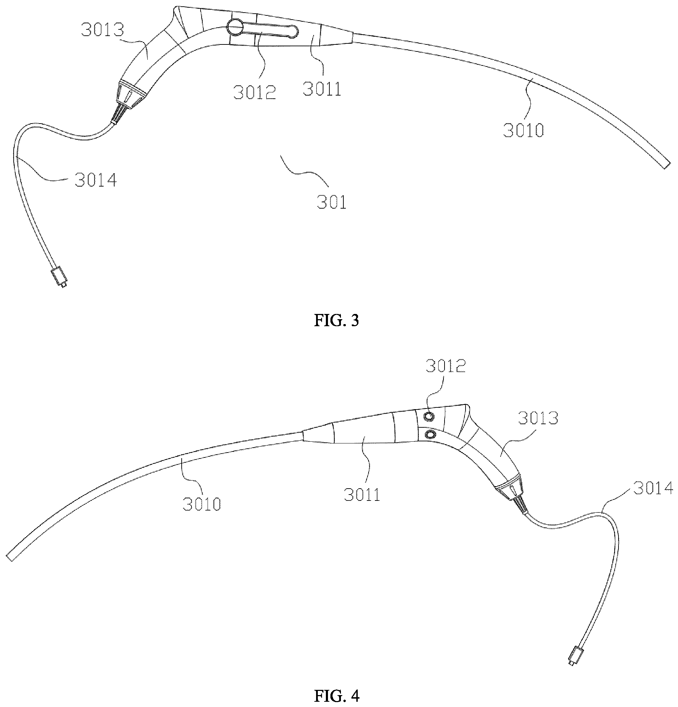

Further, as shown in FIGs. 3 to 4, the endoscope end 301 uses a disposable endoscope head, the endoscope end 301 comprises an operation part 3012 and an imaging insertion part 3010, the operation part and the imaging insertion part are connected by a detachable connection part 3011 to facilitate replacement of the imaging insertion part to adapt to different departments, the operation part 3012 is placed in an endoscope handle 3013, the operation part comprises a flexible endoscope direction control handle and an adjustment button, and a connection line 3014 at the other end of the endoscope handle transmits information acquired by the imaging insertion part. The operation part enables endoscope heads of various models and sizes to be inserted into the reusable handle end for matching. The imaging insertion part enters a lesion site in a patient body to obtain an image. The image resolution of the electronic camera in the insertion part has various specifications. The camera pixels of the insertion part are 1 million pixels (1280×720), 2 million pixels (1920x1080), 5 million pixels (2592x1944), and 8 million pixels (3288x2512). Among them, 8 million pixels reach the imaging definition of 4K optical mirrors currently on the market.

Further, to facilitate the insertion of the surgical instrument and the flushing of the insertion part, an instrument channel and a flushing channel are reserved at the top of the insertion part. To more clearly observe the pathological characteristics of the lesion of the patient, a balloon is reserved at the top of the insertion part. After the balloon is inserted into the human body, the camera field of view after the insertion part enters the human body can be improved. The balloon comprises a single balloon or a double balloon, which can be selected based on the insertion site or the physical condition of the patient. The single-balloon insertion part is characterized in that a balloon is positioned on the wall of the camera terminal tube and expands after being inflated. The double-balloon insertion part is characterized in that the balloons are positioned on the wall of the camera terminal tube and the balloon channel. After the balloons enter the human body, the balloon catheter is extended by operating the balloon assembly of the operation part. After reaching the ideal position, the front and rear balloons are inflated and then expand an observation space, and the brightness is adjusted in conjunction with the electronic light source control system to enable the physician to observe more clearly, thus obtaining a clear image of the lesion site. The digital decoder decodes and encodes the acquired image into digital signals and transmits digital signals to the image processing system through the wireless data transmission system.

Further, as shown in FIG. 1, the image processing system 40 comprises a data receiving module 400, an image processing module 401, an image display module 402, and an information entry module 403. The data receiving module is configured to receive the image data transmitted by the image acquisition system in a wireless mode. The image processing module is configured to process the image, wherein an image dehazing technology is used to improve the image processing speed, achieve the real-time dehazing effect, and achieve the vascular enhancement effect by utilizing a vascular enhancement technology.

Further, the image dehazing adopts an improved dark channel dehazing algorithm, which is specifically implemented as follows:

steps:

1) inputting a hazed image $H(x)$;
2) solving $M(x)$;

$$M(x) = \min_{c \in \{r,g,b\}} (H^c(x))$$

3) performing mean filtering on $M(x)$ to obtain $M_{ave}(x)$;

$$M_{ave}(x) = average_{s_a}(M(x))$$

4) solving an average value $m_{ave}$ of all elements in all $M(x)$;
5) solving $L(x)$ by using $M_{ave}(x)$, wherein $L(x)$ is an atmospheric transmittance, $\rho$ is a parameter used for adjustment, when the $\rho$ value is larger, the overall image is darker, and the dehazing effect is more significant, and when $\rho$ is smaller, the image is white and has an obvious haze;

$$L_o(x) = min(min(\rho m_{ave}, 0.9)M_{ave}(x), M(x))$$

6) solving $A$ by using $M_{ave}(x)$ and $H(x)$, wherein $A$ is the global atmospheric light, that is, an average value of a maximum value of all pixel components of RGB of an original image (it is estimated that 99% of which are 255) and a maximum value of the dark channel is solved; and

$$A = \frac{1}{2}(max(\max_{c \in \{r,g,b\}} (H^c(x)) + max(M_{aw}(x)))[1 \quad 1 \quad 1]^T$$

7) outputting a dehazed image $F(x)$, and solving a result value by using a standard dehazing model.

$$F(x) = \frac{H(x) - L(x)}{1 - \frac{L(x)}{A}}$$

Compared with the dark channel prior dehazing algorithm proposed by He Kaiming et al., this algorithm in the present invention mainly makes improvements in the third and seventh steps, wherein the mean blur is used in the third step. Specifically, by using the column histogram-related technology, only the pixels at the start position of each cycle need to be specially processed, and the other positions can be accumulated by simple addition and subtraction, so as to quickly achieve blurring and greatly speed up the operation speed. In the seventh step, division and floating point operation exist in the original algorithm, which can reduce the operation speed. It is observed from the formula in the seventh step that there are only two independent variables $H(x)$ and $L(x)$, and the values of the independent variables are integers between [0,255]. Therefore, if a lookup table is established in advance, the calculation amount of this lookup table is only 256×256 times, which is much less than the number of direct calculations, which will definitely increase the speed of the program. Another advantage of the number 256 is that it can be used to assist in calculating the subscript of the lookup table. By adopting the improved dark channel prior algorithm, the image processing speed is greatly improved, and the real-time dehazing effect is achieved.

The vascular enhancement technology adopts a multi-scale vascular enhancement algorithm based on a Hessian matrix. The Hessian matrix is a squared matrix of second-order partial derivatives of a scalar function, and the mixed partial derivative in x and y directions is as follows:

$$f_{xy} = f_{yx} = \frac{\partial^2 f}{\partial x \partial y} = f(x+1, y+1) + f(x,y) - f(x+1,y) - f(x,y+1)$$

An image has only x and y directions, so that the Hessian matrix of the image is a binary matrix, corresponding to:

$$G_{XX} = \frac{\partial^2 g(x,y)}{\partial x^2}$$

$$G_{yy} = \frac{\partial^2 g(x,y)}{\partial y^2}$$

$$G_{xy} = \frac{\partial^2 g(x,y)}{\partial x \partial y}$$

$$I_{XX} = G_{XX} * Img$$

$$I_{yy} = G_{yy} * Img$$

$$I_{Xy} = G_{Xy} * Img$$

$$H = \begin{bmatrix} I_x & I_{xy} \\ I_{xy} & I_{yy} \end{bmatrix}$$

For this algorithm, Frangi filter is used. For Frangi filter, there are two-dimensional and three-dimensional cases. For the image, only two-dimensional case is involved. The Frangi filtering is an edge detection enhancement filtering algorithm constructed using the Hessian matrix.

Since $f_{xy} = f_{yx}$, $H$ is a real symmetric matrix, two eigenvalues $\lambda_1$ and $\lambda_2$ are used to construct enhanced filtering, and in a two-dimensional image space, the two eigenvalues $\lambda_1$ and $\lambda_2$ of the Hessian matrix are calculated by the following formulas;

$$\lambda_1 = K + \sqrt{K^2 - Q^2}, \ \lambda_2 = K - \sqrt{K^2 - Q^2}$$

wherein:

$$K = (f_{xx} + f_{yy})/2, Q = \sqrt{f_{xx}f_{yy} - f_{xy}f_{yx}}$$

for vascular enhancement, when the diameter of the blood vessels is smaller than the width and height of the window rectangle corresponding to the current scale, the eigenvalues of the Hessian matrix of the tubular blood vessel satisfy: $|\lambda_1| \approx 0$, $|\lambda_1| << |\lambda_2|$. the eigenvalues R, S are defined by using $\lambda_1$ and $\lambda_2$:

$$R_B = \frac{|\lambda_1|}{|\lambda_2|} \quad S = \|H\|F = \sqrt{\sum_{j \leq D} \lambda_j^2}$$

at the current scale, a correspondence degree of a point p in the image belonging to the blood vessels is defined as:

$$V(\sigma, p) = \begin{cases} 0 & \text{if} \lambda_2 > 0 \text{ or } f(x,y) < f_t \\ \exp\left(-\frac{R_B^2}{2\beta^2}\right) & \exp\left(-\frac{2c^2}{\lambda_2^2}\right)\left(1 - \exp\left(-\frac{S^2}{2\gamma^2}\right)\right) \end{cases}$$

the black background in the image is directly removed by $f(x, y) < f_t$, so that the subsequent mask background interference operation is omitted, β is used to adjust differences between linear objects and block objects, c and γ are parameters that control the overall smoothness of linear objects, the range of adjustment parameters of a linear response function is $\beta \in [0.3, 2]$, $c \in [10^{-5}, 10^{-6}]$, $\gamma \in [3, 6]$, the half-width of the rectangular window of each pixel point p is generally 3σ, when the windows corresponding to different scales match the diameter of the blood vessels, $v(p)$ will get the maximum response, the response function of a point belonging to the blood vessel region at multiple scales is:

$$v(p) = \max_{\sigma \in [\sigma_{\min}, \sigma_{\max}]} V(\sigma, p)$$

through the above model calculation, the linear objects in the image, namely the blood vessels, are strengthened, thereby achieving the effect of vascular enhancement.

The information processing module comprises patient information entry, and patient detection information entry and storage. Except for the name, all other patient information entry items are optional, including age, gender, ethnicity, native place, marital status, ID number, telephone number, address, fee type, fee, examination physician, examination number, patient number, previous examination number, and inpatient number. The patient detection information entry comprises five parts, namely a photographed image for examination, symptoms, description, diagnosis and suggestion, wherein the information of other parts except the diagnosis can support quick input. The examination information can automatically form an examination report for easy printing and archiving.

The flexible and rigid endoscope system can be applied to various departments such as ventriculoscopes, otolaryngoscopes, stomatoscopes, bronchoscopes, thoracoscopes, laparoscopes, gastroscopes, arthroscopes, hysteroscopes, colonoscopes, cystoscopes, nephroscopes, duodenoscopes, and proctoscopes to acquire image data of lesion sites and process the image data.

An electronic flexible and rigid endoscope method applicable to general practice according to a preferred embodiment of the present invention, as shown in FIG. 2, comprises the following steps:

S10: acquiring image data of a lesion site by adopting the above system; and
S20: processing the image data by adopting the above system, wherein the specific method is the same as the above steps and is not repeated here.

**Claims**

1. An electronic flexible and rigid endoscope system applicable to general practice, comprising: a data acquisition system, configured to acquire image data of a lesion site, and a data processing system, configured to process the image data from the data acquisition system, wherein the data acquisition system comprises a handle end and an endoscope end, the endoscope end comprises an imaging insertion end and an operating end, the imaging insertion end enters the lesion site to acquire images of the lesion site, the operating end controls the action of the imaging insertion end, the handle end sends the image data acquired by the endoscope end to the data processing system, the data processing system comprises a data receiving module and an image processing module, the data receiving module receives the image data acquired by the data acquisition system, and the image processing module performs dehazing through a dark channel prior algorithm and performs vascular enhancement through a multi-scale vascular enhancement algorithm.

2. The system according to claim 1, wherein the dark channel prior algorithm processes pixels at a start position of each cycle by using an average blur value in the mean filtering process, processes pixels at other positions by using an accumulated sum, and simultaneously establishes a 256×256 lookup table in the process of outputting the dehazed image to improve an image processing speed.

3. The system according to claim 1, wherein the multi-scale vascular enhancement algorithm enhances blood vessels in a two-dimensional image by using a Frangi filter based on a Hessian matrix.

4. The system according to claim 1, wherein the handle end is provided with a lens digital decoder and an electronic light source control system, the lens digital decoder converts the acquired image into a digital signal image, the electronic light source control system provides brightness for the data acquisition system, the imaging insertion part is further connected to an instrument channel and a cleaning channel, and the data processing system further comprises an image display module and an information input module.

5. The system according to claim 4, wherein an end of the imaging insertion part is provided with a balloon, the balloon is a single balloon or a double balloon, the single balloon is positioned on a tube wall of the data acquisition system, and the double balloon is positioned on a tube wall of the data acquisition system and a balloon channel.

6. An electronic flexible and rigid endoscope method applicable to general practice, comprising the following steps: acquiring image data of a lesion site, processing the acquired image data, wherein dehazing is performed through a dark channel prior algorithm, and vascular enhancement is performed through a multi-scale vascular enhancement algorithm.

7. The method according to claim 6, wherein the step of performing dehazing through a dark channel prior algorithm comprises:

step I: inputting a hazed image $H(x)$;
step II: solving $M(x)$;

$$M(x) = \min_{c \in \{r,g,b\}} (H^c(x))$$

step III: performing mean filtering on $M(x)$ to obtain $M_{ave}(x)$;

$$M_{ave}(x) = average_{s_a}(M(x))$$

step IV: solving an average value $m_{ave}$ of all elements in all $M(x)$;
step V: solving $L(x)$ by using $M_{ave}(x)$, wherein $L(x)$ is an atmospheric transmittance, $\rho$ is a parameter used for adjustment, when the p value is larger, the overall image is darker, and the dehazing effect is more significant, and when $\rho$ is smaller, the image is white and has an obvious haze;

$$L_o(x) = min(min(\rho m_{ave}, 0.9)M_{ave}(x), M(x))$$

step VI: solving $A$ by using $M_{ave}(x)$ and $H(x)$, wherein $A$ is the global atmospheric light, that is, an average value of a maximum value of all pixel components of RGB of an original image and a maximum value of the dark channel is solved; and

$$A = \frac{1}{2}(max(\max_{c \in \{r,g,b\}} (H^c(x)) + max(M_{aw}(x)))[1 \quad 1 \quad 1]^T$$

step VII: outputting a dehazed image F(x), and solving a result value by using a standard dehazing model.

$$F(x) = \frac{H(x) - L(x)}{1 - \frac{L(x)}{A}}$$

8. The method according to claim 7, wherein in the step III, pixels at a start position of each cycle are specially processed by using mean blur, and pixels at other positions are processed by using an accumulated sum to rapidly achieve blur; and
in the step VII, a 256×256 lookup table is established, and the image processing speed is increased by shifting to assist in calculating the subscript of the lookup table for real-time dehazing.

9. The method according to claim 6, wherein the performing vascular enhancement through a multi-scale vascular enhancement algorithm to achieve image processing comprises: a Hessian matrix of a squared matrix of second-order partial derivatives of a scalar function, the mixed partial derivative in x and y directions is as follows:

$$f_{xy} = f_{yx} = \frac{\partial^2 f}{\partial x \partial y} = f(x+1, y+1) + f(x,y) - f(x+1,y) - f(x,y+1)$$

Hessian binary matrices corresponding to the image in x and y directions are as follows:

$$G_{XX} = \frac{\partial^2 g(x,y)}{\partial x^2}$$

$$G_{yy} = \frac{\partial^2 g(x,y)}{\partial y^2}$$

$$G_{xy} = \frac{\partial^2 g(x,y)}{\partial x \partial y}$$

$$I_{XX} = G_{XX} * Img$$

$$I_{yy} = G_{yy} * Img$$

$$I_{Xy} = G_{Xy} * Img$$

$$H = \begin{bmatrix} I_x & I_{xy} \\ I_{xy} & I_{yy} \end{bmatrix}$$

**10.** The method according to claim 9, wherein a Frangi filter is used in the Hessian matrix, since $f_{xy} = f_{yx}$, $H$ is a real symmetric matrix, two eigenvalues $\lambda_1$ and $\lambda_2$ are used to construct enhanced filtering, and in a two-dimensional image space, the two eigenvalues $\lambda_1$ and $\lambda_2$ of the Hessian matrix are calculated as follows;

$$\lambda_1 = K + \sqrt{K^2 - Q^2} \; , \; \lambda_2 = K - \sqrt{K^2 - Q^2}$$

wherein:

$$K = (f_{xx} + f_{yy})/2, Q = \sqrt{f_{xx}f_{yy} - f_{xy}f_{yx}}$$

for vascular enhancement, when the diameter of the blood vessels is smaller than the width and height of the window rectangle corresponding to the current scale, the eigenvalues of the Hessian matrix of the tubular blood vessel satisfy: $|\lambda 1| \approx 0$, $|\lambda 1| << |\lambda 2|$, the eigenvalues R, S are defined by using $\lambda 1$ and $\lambda 2$:

$$R_B = \frac{|\lambda_1|}{|\lambda_2|} \quad S = \|H\|F = \sqrt{\sum_{j \leq D} \lambda_j^2}$$

at the current scale, a correspondence degree of a point p in the image belonging to the blood vessels is defined as:

$$V(\sigma, p) = \begin{cases} 0 & \text{if}\lambda_2 > 0 \text{ or } f(x,y) < f_t \\ \exp\left(-\dfrac{R_B^2}{2\beta^2}\right) & \exp\left(-\dfrac{2c^2}{\lambda_2^2}\right)\left(1 - \exp\left(-\dfrac{S^2}{2\gamma^2}\right)\right) \end{cases}$$

the black background in the image is directly removed by $f(x, y) < f_t$, so that the subsequent mask background interference operation is omitted, β is used to adjust differences between linear objects and block objects, c and $\gamma$ are parameters that control the overall smoothness of linear objects, the range of adjustment parameters of a linear response function is $\beta \in [0.3,2]$, $c \in [10^{-5}, 10^{-6}]$, $\gamma \in [3,6]$, the half-width of the rectangular window of each pixel point p is generally 3 $\sigma$, when the windows corresponding to different scales match the diameter of the blood vessels, $v(p)$ will get the maximum response, the response function of a point belonging to the blood vessel region at multiple scales is:

$$v(p) = \max_{\sigma \in [\sigma_{\min}, \sigma_{\max}]} V(\sigma, p)$$

through the above model calculation, the linear objects in the image, namely the blood vessels, are strengthened, thereby achieving the effect of vascular enhancement.

S10

Acquiring image data of a lesion site

S20

Processing image data through an image dehazing technology and a vascular enhancement technology, wherein real-time dehazing is performed through a dark channel dehazing algorithm, and blood vessels are enhanced through a multi-scale vascular enhancement algorithm, so that image processing is achieved

FIG. 1

40

| Image acquisition system | | Image processing system | 400 |
| --- | --- | --- | --- |
| 30 | | | |
| | Handle end | Data receiving module | 401 |
| 300 | | Image processing module | 402 |
| | Endoscope end | Image display module | 403 |
| 301 | | Information entry module | |

FIG. 2

3013

3012 3011

3010

3014

301

**FIG. 3**

3012

3013

3010 3011

3014

**FIG. 4**

Europäisches
Patentamt
European
Patent Office
Office européen
des brevets

# EUROPEAN SEARCH REPORT

**Application Number**

EP 24 20 8032

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 116 777 786 A (HANGZHOU HAIKANG HUIYING TECH CO LTD) 19 September 2023 (2023-09-19) * figures 1, 2, 5 * ----- | 1-10 | INV. A61B1/00 G06T5/20 G06T5/73 |
| A | CN 114 881 896 A (GUANGDONG OPTOMEDIC TECH CO LTD) 9 August 2022 (2022-08-09) * figure 9 * ----- | 1-10 | |

**TECHNICAL FIELDS SEARCHED (IPC)**

A61B
G06T

The present search report has been drawn up for all claims

| Place of search | Date of completion of the search | Examiner |
|---|---|---|
| Munich | 17 March 2025 | Streif, Jörg Ulrich |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 20 8032

This annex lists the patent family members relating to the patent documents cited in the above-mentioned European search report.
The members are as contained in the European Patent Office EDP file on
The European Patent Office is in no way liable for these particulars which are merely given for the purpose of information.

17-03-2025

| Patent document cited in search report | | Publication date | Patent family member(s) | Publication date |
|---|---|---|---|---|
| CN 116777786 | A | 19-09-2023 | NONE | |
| CN 114881896 | A | 09-08-2022 | NONE | |

EPO FORM P0459

For more details about this annex : see Official Journal of the European Patent Office, No. 12/82